Europäisches Patentamt

European Patent Office

Office européen des brevets

(11) Publication number: **0 344 530**
**A1**

# EUROPEAN PATENT APPLICATION

(21) Application number: **89108948.4**

(22) Date of filing: **18.05.89**

(51) Int. Cl.⁴: **A61M 25/00**

(30) Priority: **27.05.88 US 199940**

(43) Date of publication of application:
**06.12.89 Bulletin 89/49**

(84) Designated Contracting States:
**CH DE FR GB IT LI NL**

(71) Applicant: **ADVANCED CARDIOVASCULAR
SYSTEMS, INC.**
**3200 Lakeside Drive**
**Santa Clara California 95052(US)**

(72) Inventor: **Miller, Gary Henry**
**529 Bryce Court**
**Milpitas, CA 95035(US)**

(74) Representative: **Baillie, Iain Cameron et al**
**c/o Ladas & Parry Isartorplatz 5**
**D-8000 München 2(DE)**

(54) Vascular catheter assembly with a guiding sleeve.

(57) Dilatation catheter assembly particularly suitable for use in valvuloplasty procedures. A plurality of dilatation catheters each having an inflatable balloon and a short guide sleeve in the distal portion thereof are introduced and advanced individually along a guidewire into the cardiovascular system of a patient. The catheters are introduced individually so that a smaller incision with less trauma to the patient can be used. Once inside the cardiovascular system, the catheters can be manipulated to position the balloons side by side in an area such as an aortic valve where the combined dilatation effect of a plurality of balloons can be advantageously employed.

FIG. 1

## VASCULAR CATHETER ASSEMBLY WITH A GUIDING SLEEVE

### Background of the Invention

This invention pertains, generally to balloon dilatation catheters, and more particularly to a dilatation catheter assembly and method which are particularly suitable for use in valvuloplasty.

In recent years, dilatation catheters with inflatable balloons have come into increasingly wide use of treating stenotic lesions in the coronary arteries. The catheters are most commonly introduced percutaneously into the cardiovascular system through the femoral or brachial arteries with local anesthesia. The dilatation catheter is typically introduced through a guiding catheter and advanced along a guidewire until the balloon traverses or crosses the lesion. The balloon is then inflated to compress the atherosclerosis in a direction generally perpendicular to the wall of the artery, thereby dilating the lumen of the artery.

More recently, it has been proposed to use dilation catheters in other portions of the coronary anatomy such as the mitral valve. This procedure is known as mitral stenosis valvuloplasty.

With procedures such as mitral stenosis valvuloplasty, a relatively large amount of dilatation is required. However, balloons which are large enough to provide the requisite amount of dilatation can be difficult to introduce percutaneously and can increase the trauma to which a patient is exposed to an undesirable level.

What has been needed, but heretofore unavailable, is a dilatation catheter system which provides sufficient dilatation for valvuloplasty, but which can be easily percutaneously introduced. The present invention satisfies that need.

### Summary of the Invention

The present invention provides a dilatation catheter assembly comprising a plurality of dilatation catheters each having an elongated tubular member with proximal and distal portions, an inflatable balloon on the distal portion of the tubular member, a longitudinally extending passageway in the tubular member communicating with the interior of the balloon for inflation and deflation thereof and a relatively short guide sleeve in the distal portion of the tubular member distal to the balloon which is adapted to slidably receive a guidewire, the balloons disposed on the tubular member so that a plurality of catheters can be sequentially mounted on a guidewire and with the balloons so positioned laterally adjacent to each other.

The dilatation catheters can be percutaneously introduced and separately advanced along a guidewire disposed within a patient's arterial system. The catheters are introduced one at a time and advanced individually along the guidewire to position the balloons so that they are adjacent to one another in the mitral valve or other portion of the cardiovascular system where dilatation is required. By sliding a plurality of catheters over the guidewire and positioning the balloons side by side, there can be sufficient amounts of dilatation for effective valvuloplasty. These and other advantages will become more apparent from the following detailed description of the invention when taken in conjunction with the accompanying exemplary drawings.

### Brief Description of the Drawings

Fig. 1 is a side elevational view of one embodiment of a dilatation catheter assembly with a plurality of dilatation catheters nested in position on a guidewire according to the invention;

Fig. 2 is a cross-sectional view taken along line 2-2 in Fig. 1; and

Fig. 3 is an enlarged fragmentary elevational view partly broken away, of one of the nested catheters shown in Fig. 1.

### Detailed Description of the Invention

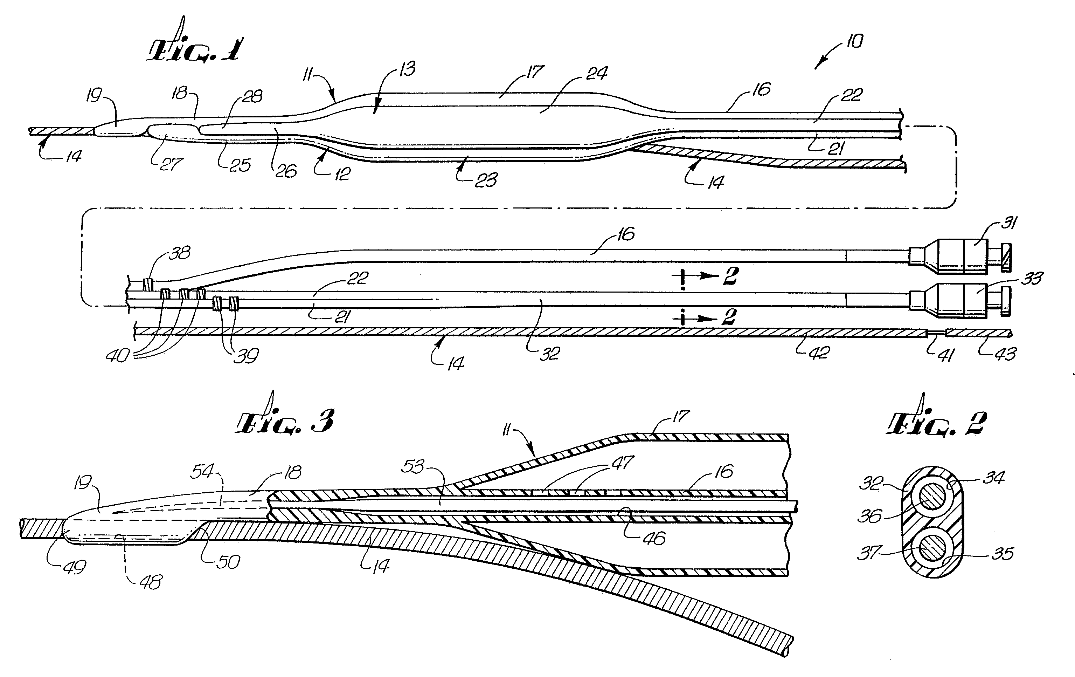

Fig. 1 illustrates a catheter assembly 10 embodying features of the invention, which includes a plurality of dilatation catheters 11-13 slidably disposed on a guidewire 14. Three catheters are provided and they are spaced equally at angles on the order of 120° about the guidewire. It will be understood, however, that a greater or lesser number of catheters can be employed.

The distal portions of catheters 11-13 are of substantially similar structure. Catheter 11, which is exemplary, comprises an elongated tubular member 16, an inflatable balloon 17 on the distal portion of tubular member 16 and a distal tip portion 18 which extends distally from the balloon. A relatively short guide sleeve 19 is slidably mounted about guidewire 14 and is positioned off-axis with respect to the longitudinal axis of the catheter. Similarly, catheters 12 and 13 have respectively elongated tubular shafts 21 and 22, inflatable balloons 23 and 24, distal tip portions 25 and 26 and guide sleeves 27 and 28. The passageways through the guide sleeves 19, 27, and 28 are sufficient in diameter to allow the guidewire 14 to pass freely therethrough so that the catheters can be easily advanced through or withdrawn from the patient's vasculature along the guidewire 14. Preferably, the longitudinal axis of the guide sleeves are spaced from parallel or near parallel with the longitudinal axis of the respective catheters. The distal tip portions 18, 25, and 26 of the catheters are of different lengths so that the balloons can be positioned side by side, as illustrated, with the guide sleeves nested sequentially against one another on the guidewire 14.

A Luer fitting 31 is connected to the proximal end of the tubular member 16 of catheter 11 for connection to a source of pressurized fluid (not shown) for inflation and deflation of balloon 17. The tubular members 21, 22 have a common proximal section or shaft 32 (see Fig. 2) having a Luer fitting 33 at its proximal end for connection to a source (not shown) of pressurized fluid for inflation/deflation of balloons 22, 27. As illustrated in Fig. 2, shaft 32 has a pair of longitudinally extending lumens 34 and 35 positioned side by side therein. These lumens communicate with the respective lumens in the tubular members 21 and 22 of catheters 12, 13, and they communicate with a common passageway in Luer fitting 33. Stiffener members 36 and 37 are disposed within lumens 34 and 35, respectively.

The dual lumen tubular member 32 has been found to make the catheter assembly 10 easier to use in that it reduces the number of shafts which the doctor must handle. Also, the common shaft 32 of the two following catheters 12 and 13 has a distinctively different feel from the single shaft 16 of the lead catheter 11. The common shaft 32 does, however, impose some limitation on the independent movement of the two following catheters, and in some applications, it may be preferable to eliminate the common shaft and employ independent catheters. In other applications, however, it may be preferable to connect all three of the catheters to a common shaft. With an assembly having different numbers of catheters, any desired number of the catheters can be connected to a common shaft. While a plurality of catheters are contemplated in the presently preferred embodiments for mitral valvuloplasty, one catheter can be employed particularly for angioplasty procedures.

Markers 38, 39, and 40 are provided for identifying the three catheters from the proximal ends of the catheters. In the embodiment illustrated, marker 38 comprises a single colored band on the shaft 16 of the lead catheter 11 i.e.. the catheter having its guide sleeve positioned most distally on guidewire 14. Marker 39 comprises two colored bands on the shaft 21 of the second catheter 12 and marker 40 comprises three colored bands on the shaft 26 of the third catheter 13.

In the embodiment illustrated in Fig. 1, guidewire 14 has an elongated wire core 41 which extends coaxially within an outer coil 42. The distal end portions of the guidewire core and coil are affixed or welded together by suitable means such as soldering, brazing (not shown), and a control knob 43 is connected to the proximal end of core 41 to rotate the core and facilitate the steering of the guidewire to the desired location within a patient's vasculature. This type of guidewire is sometimes referred to as a movable core guidewire, but it will be understood that the invention is not limited to use with this particular type of guidewire. Any other suitable type of guidewire, such as a fixed guidewire, can be employed, if desired.

The structure of one of the three catheters, namely, catheter 11, is shown in more detail in Fig. 3. The other two catheters 12 and 13 are of substantially identical construction except for the lengths of their distal tip portions and the presence of dual lumen shaft 32.

As illustrated in Fig. 3, shaft 16 comprises an elongated tubular member which extends through balloon 17 and has an axially extending lumen 46. The proximal and distal end portions of the balloon 17 are sealably secured to the sidewall of the tubular member 16, and openings 47 are provided in the sidewall to connect in fluid communication the lumen 46 and the interior of the balloon. The balloon 17 can be formed as part of the tubular member 16, as illustrated, or they can be formed separately and bonded together by suitable means such as heat sealing or adhesive bonding.

Guide sleeve 19 comprises a relatively short cylindrical body having a passageway 48 therein through which guidewire 14 can pass freely. The sleeve 19 has a length on the order of about 0.5 to about 5 cm, and the leading or distal end 49 of the shoe or sleeve is rounded and tapered to facilitate insertion into the patient's vascular system. The trailing or proximal end 50 of the shoe or sleeve 19 is beveled to receive the leading end of the sleeve 27 in a closely nestled relationship.

A stiffening member or wire 53 may be provided to extend axially within the lumen 46 and into distal rigidity or "pushability" if needed. The distal end portion 54 of the stiffener 53 is affixed to the distal tip portion 18 of the catheter by suitable means such as heat sealing or adhesive bonding and is preferably tapered to provide a greater flexibility thereto.

The tubular members and tip portions of the catheters are preferably fabricated from a high density polyethylene material, the balloons are fabricated of an irradiated polyethylene or polyethylene terephthalate, and the guidewire and stiffening wires are fabricated of stainless steel. e.g.. 304 type.

For mitral stenosis valvuloplasty each of the balloons typically have a diameter of about 8-27 mm, and each of the shafts of the three catheters typically have outer diameter of about .040 - .042 inch (1.016 - 1.067 mm) and an inner diameter of about 0.034 - 0.036 inch (0.864 - 0.914 mm). The stiffening wires have a diameter on the order of 0.016 inch, tapering down to a diameter of .010 inch (0.254 mm) over about 2.5 cm at the distal ends of the wires. The lumens and dual lumen shaft 32 each have a diameter on the order of 0.034 - 0.036 inch (0.864 - 0.914 mm), and this latter shaft has a width on the order of .083 - .085 inch (2.108 - 2.159 mm) and a thickness on the order of 0.040 - 0.042 inch (1.016 -1.067 mm). The catheters typically have an overall length of about 90-160 cm, and dual lumen shaft 32 can extend as far as about 65% of the length of the shafts. The passageway 48 has a diameter of about 0.040 - 0.042 inch (1.016 - 1.067 mm). Guidewire 14 has an outer diameter of about .038 inch (0.965 mm).

The catheter assembly of the invention is typically used in the following manner. First, guidewire 14 is introduced percutaneously into the cardiovascular system of a patient through a suitable introducer (not shown). The catheters are then introduced over the guidewire one at a time, beginning with lead catheter 11 and followed by catheters 12 and 13. The guide shoe or sleeve of each successive catheter is slipped over the proximal end of the guidewire outside the body of the patient. The catheters are introduced so that only one balloon passes through the introducer at a time. Once the balloons are inside the cardiovascular system, the shafts can be manipulated to position the balloons side by side in the mitral valve or other area where dilation is to occur. Dilatation is effected by pressurizing the balloons to inflate them simultaneously in the side-by-side position. When the dilatation procedure is complete, the balloons are deflated and then catheters are withdrawn individually, starting with trailing catheter 13. The proper sequence for introduction and withdrawal of the catheters is indicated by the markers 38-40 toward the proximal ends of the shafts.

The invention has a number of important features and advantages. The combined dilatation provided by the balloons is substantially greater than the dilatation which would be possible with a single balloon of similar size. Since the balloons are individually movable along the guidewire, they can be inserted and withdrawn one at a time., minimizing the size of the incision and the trauma to the patient. In addition, the disclosed embodiment with the three balloons while described herein for mitral valve dilatation is also suitable for use in dilating aortic valves. A single catheter design may be employed for angioplasty procedure.

It is apparent from the foregoing that a new and improved dilatation catheter assembly and method of using the same have been provided. While the invention has been described herein in terms of dilatation balloon type catheters, the invention may also be used with diagnostic type catheters without a balloon with an off-axis shoe or sleeve which is adapted to be slidably mounted about a guidewire. In this embodiment, suitable sensors could be placed at the distal or proximal ends, such as pressure or fluid flow sensors. In the latter instance, the inner lumen of the catheter would have an opening at the distal end of the catheter. It will be apparent to those familiar with the art that other modifications and improvements can be made without departing from the scope of the invention.

## Claims

1. A dilatation catheter assembly comprising a plurality of dilatation catheters each having an elongated tubular member with proximal and distal portions, an inflatable balloon on the distal portion of the tubular member, a longitudinally extending passageway in the tubular member communicating with the interior of the balloon for inflation and deflation thereof and a relatively short guide sleeve in the distal portion of the tubular member distal to the balloon which is adapted to slidably receive a guidewire, the balloons disposed on the tubular member so that a plurality of catheters can be sequentially mounted on a guidewire and with the balloons so positioned laterally adjacent to each other.

2. The catheter assembly of claim 1 wherein three dilatation catheters are provided, and the balloons are positioned approximately 120o apart about a common axis.

3. The catheter assembly of claim 2 including a double lumen shaft connected to the proximal ends of the tubular members of two of the catheters, with the tubular member of the third catheter extending to the proximal end of the shaft.

4. The catheter assembly of claim 3 wherein the guide sleeve of the third catheter is positioned distally of the guide sleeves of the other two catheters on the guidewire.

5. The catheter assembly of any one of the preceding claims wherein each of the catheters has a stiffening wire extending axially within the longitudinally extending passageway and affixed to the tip portion of the catheter.

6. The catheter assembly of claim 5 wherein the distal end portion of each of the stiffening wires extends substantially the entire length of the tip portion and decreases in stiffness between the proximal and distal ends of the tip portion.

7. The catheter assembly of any one of the preceding claims wherein the catheters are provided with distal tip portions of differing lengths so that the guide sleeves nest sequentially on the guidewire when the balloons are in a desired side-by-side position within a patient's cardiovascular system.

8. A catheter for performing medical procedures within a patient's vasculature having an elongated tubular member with proximal and distal portions and a guide sleeve of relatively short length disposed off-axis from the longitudinal axis of the catheter and having a passageway therein adapted to slidably receive a guidewire.

9. The catheter of claim 8 wherein an inflatable balloon is provided on the distal portion proximally of the guide sleeve and means are provided on the proximal end of the tubular member to connect the tubular member to a source for inflation fluid for the balloon.

10. The catheter of claim 8 wherein at least one sensor is provided on the distal portion of the tubular member.

*Fig. 1*

*Fig. 3*

*Fig. 2*

EP 0 344 530 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CH-A- 654 214 (SCHNEIDER MEDINTAG AG) --- | | A 61 M 29/02<br>A 61 M 25/00 |
| A | US-A-2 687 131 (RAICHE) ----- | | |

TECHNICAL FIELDS SEARCHED (Int. Cl.4)

A 61 M

The present search report has been drawn up for all claims

| Place of search | Date of completion of the search | Examiner |
|---|---|---|
| THE HAGUE | 26-09-1989 | MIR Y GUILLEN V. |